# EUROPEAN PATENT APPLICATION

(11) **EP 4 109 074 A1**
(43) Date of publication of application: **28.12.2022**
(21) Application number: 21756875.7
(22) Date of filing: 02.02.2021
(51) Int. Cl.: G01N 15/12, C12M 1/34, G01N 27/00

(54) **PARTICLE ANALYSIS DEVICE**

(30) Priority: 20.02.2020 JP 2020026876
(71) Applicant: NOK Corporation, Minato-ku, Tokyo 105-8585 (JP)
(72) Inventor: YOSHITOMI, Takumi, Fujisawa-shi, Kanagawa 251-0042 (JP); MUROTA, Yuki, Fujisawa-shi, Kanagawa 251-0042 (JP); FUJISAWA, Naohiro, Fujisawa-shi, Kanagawa 251-0042 (JP); SATO, Kosuke, Fujisawa-shi, Kanagawa 251-0042 (JP); TOKI, Yuto, Fujisawa-shi, Kanagawa 251-0042 (JP)
(74) Representative: Global IP Europe Patentanwaltskanzlei
(86) International application number: PCT/JP2021/003674
(87) International publication number: WO 2021/166629

(57) **Abstract**

A particle analysis device includes multiple stacked plates joined together; an upper liquid space adapted to store a first liquid; a lower liquid space adapted to store a second liquid; a connection pore connecting the upper liquid space to the lower liquid space; a first hole extending from the top surface to the upper liquid space, the first liquid flowing through the first hole; and a second hole extending from the top surface to the lower liquid space, the second liquid flowing through the second hole. A first electrode and a second electrode that are sheets are pinched between two of the plates. The first electrode applies an electric potential to the first liquid in the upper liquid space through the first hole, whereas the second electrode applies an electric potential to the second liquid in the lower liquid space through the second hole. The particle analysis device further includes a first electrode-rod-insertion hole extending from the top surface to the first electrode, and a second electrode-rod-insertion hole extending from the top surface to the second electrode. The first electrode and the second electrode are not exposed at any side surface of the particle analysis device.

## Description

### TECHNICAL FIELD

The present invention relates particle analysis devices for analyzing particles contained in a liquid.

### BACKGROUND ART

A particle analysis device having two spaces has been proposed for analyzing particles, such as exosomes, pollens, viruses, and bacteria (Patent Documents 1-4). This type of particle analysis device has a pore connecting the two spaces, in which a liquid is stored in one space and another liquid containing particles to be analyzed is stored in the other space. These spaces are provided with different electrical potentials for causing electrophoresis, so that particles pass through the pore. As the particles pass through the pore, the current value flowing through the liquid changes. By observing the change in the current value, characteristics (e.g., type, shape, and size) of the particles that passed through the pore can be analyzed. For example, it is possible to determine the number of particles of a certain type contained in the liquid.

### BACKGROUND DOCUMENT(S)

### Patent Document(s)

Patent Document 1: JP-A-2014-174022
Patent Document 2: JP-A-2017-156168
Patent Document 3: WO 2013/13430 A
Patent Document 4: WO 2013/137209 A

### SUMMARY OF THE INVENTION

In the use of this type of particle analysis device, it is desired to improve the certainty of particle analysis. In particular, it is desired to prevent a short circuit between two electrodes used to cause electrophoresis and to measure current values.

Accordingly, the present invention provides a particle analysis device capable of preventing or reducing a short circuit between two electrodes.

According to an aspect of the present invention, there is provided a particle analysis device including multiple stacked plates joined together; an upper liquid space formed in at least one of the plates and adapted to store a first liquid; a lower liquid space formed in at least one of the plates, disposed below the upper liquid space and adapted to store a second liquid; a connection pore disposed in at least one of the plates and connecting the upper liquid space to the lower liquid space; a first hole having an opening that opens at a top surface of the particle analysis device, the first hole penetrating at least one of the plates and extending from the top surface to the upper liquid space, the first liquid flowing through the first hole; a second hole having an opening that opens at the top surface, the second hole penetrating at least two of the plates and extending from the top surface to the lower liquid space, the second liquid flowing through the second hole; a first electrode that is a sheet pinched between two of the plates and adapted to apply an electric potential to the first liquid in the upper liquid space through the first hole; a second electrode that is a sheet pinched between the two of the plates, between which the first electrode is pinched, and adapted to apply an electric potential to the second liquid in the lower liquid space through the second hole; a first electrode-rod-insertion hole having an opening that opens at the top surface, the first electrode-rod-insertion hole penetrating at least one of the plates and extending from the top surface to the first electrode; and a second electrode-rod-insertion hole having an opening that opens at the top surface, the second electrode-rod-insertion hole penetrating at least one of the plates and extending from the top surface to the second electrode. The first electrode and the second electrode are configured not to be exposed at a side surface of the particle analysis device.

In this aspect, the first electrode, which applies an electric potential to the first liquid, and the second electrode, which applies an electric potential to the second liquid, are pinched between the same two plates and are arranged at the same height level. A first electrode is inserted into the first electrode-rod-insertion hole and is brought into contact with the first electrode, and second electrode is inserted into the second electrode-rod-insertion hole and is brought into contact with the second electrode. Each of the first electrode-rod-insertion hole and the second electrode-rod-insertion hole extends from the top surface of the particle analysis device to the corresponding electrode and does not open at any side surface of the particle analysis device, and the first electrode and the second electrode are not exposed at any side surface of the particle analysis device. Accordingly, in a case in which the liquid leaks from the inside of the first hole and flows along the first electrode between the two plates, even if the leaking liquid may enter the first electrode-rod-insertion hole surrounded by walls of the plates, the leaking liquid is unlikely to leak from side surfaces of the particle analysis device. Therefore, if the distance between the first electrode and the second electrode is sufficiently large and the distance between the first electrode-rod-insertion hole and the second-electrode-rod insertion hole is sufficiently large, the liquid, which leaked from the inside of the first hole, is less likely to cause a short circuit between the first electrode and the second electrode.

On the other hand, in a case in which the liquid leaks from the inside of the second hole and flows along the second electrode between the two plates, even if the leaking liquid may enter the second electrode-rod-insertion hole surrounded by walls of the plates, the leaking liquid is unlikely to leak from side surfaces of the particle analysis device. Therefore, if the distance between the first electrode and the second electrode is sufficiently large and the distance between the first electrode-rod-insertion hole and the second-electrode-rod insertion hole is sufficiently large, the liquid, which leaked from the inside of the first hole, is less likely to cause a short circuit between the first electrode and the second electrode.

Thus, according to the aspect, it is possible to improve the certainty of particle analysis.

### BRIEF DESCRIPTION OF THE DRAWINGS

FIG. 1 is a perspective view showing a particle analysis device according to an embodiment of the present invention;
FIG. 2 is a side view of the particle analysis device shown in FIG. 1;
FIG. 3 is a plan view of the particle analysis device of FIG. 1;
FIG. 4 is a conceptual diagram showing the principle of particle analysis used in the particle analysis device of FIG. 1;
FIG. 5 is an exploded view of the particle analysis device shown in FIG. 1 seen from diagonally above;
FIG. 6 is an enlarged plan view of the particle analysis device of FIG. 1;
FIG. 7 is a cross-sectional view taken along line VII-VII in FIG. 6;
FIG. 8 is an enlarged plan view, which is similar to FIG. 3, of the particle analysis device, showing liquids that leak from holes for storing the liquids;
FIG. 9 is an enlarged perspective view of the particle analysis device, showing liquids that leak from holes for storing the liquids;
FIG. 10 is a plan view of a particle analysis device according to a modification of the embodiment of the present invention;
FIG. 11 is a plan view of a particle analysis device according to another modification of the embodiment of the present invention;
FIG. 12 is a plan view of a particle analysis device according to another modification of the embodiment of the present invention;
FIG. 13 is an enlarged plan view of a particle analysis device according to another modification of the embodiment of the present invention; and
FIG. 14 is an enlarged plan view of a particle analysis device according to another modification of the embodiment of the present invention.

### DESCRIPTION OF EMBODIMENTS

Hereinafter, with reference to the accompanying drawings, various embodiments according to the present invention will be described. It is of note that the drawings are not necessarily to scale, and certain features may be exaggerated or omitted.

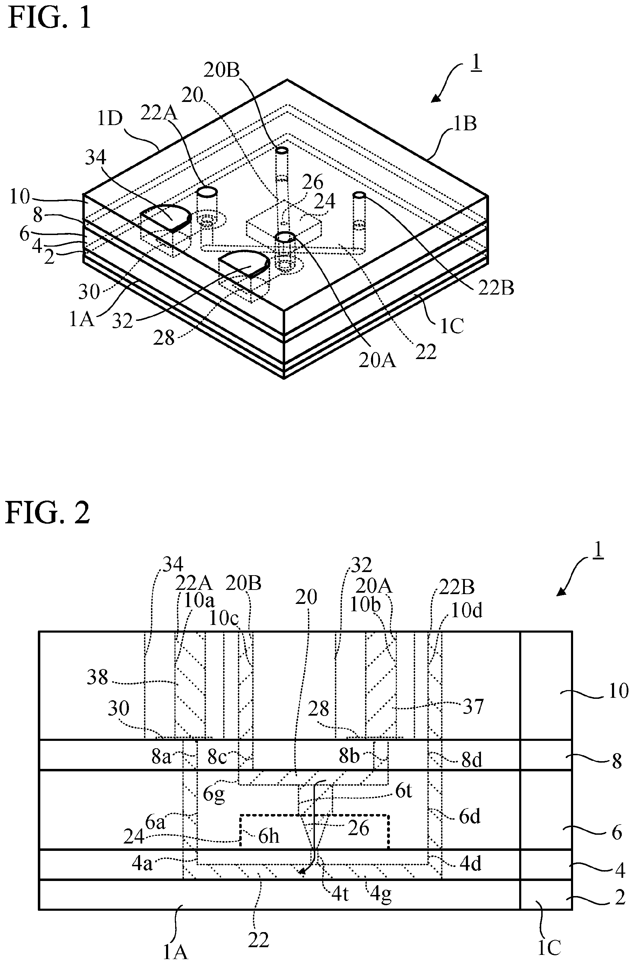

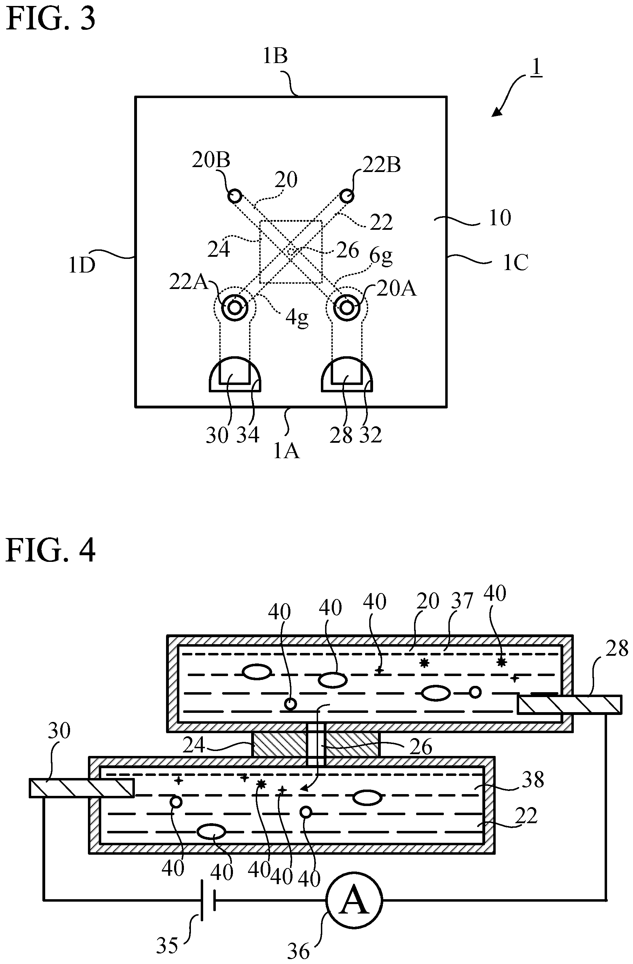

As shown in FIG. 1, a particle analysis device 1 according to an embodiment has a substantially rectangular parallelepiped shape, and the lengths of the four side surfaces 1A, 1B, 1C, and 1D are equal. That is, as shown in the plan view of FIG. 3, the particle analysis device 1 has a substantially square contour. FIG. 2 is a side view of the particle analysis device 1 showing two side surfaces 1A and 1C.

As shown in FIGS. 1, 2, and 3, the particle analysis device 1 has an upper liquid space 20, a lower liquid space 22, and a connection pore 26. Each of the liquid spaces 20 and 22 extends linearly in a horizontal direction, in which a liquid 37 is stored in the first liquid space 20 and a liquid 38 is stored in the lower liquid space 22. In FIG. 2, the liquid (first liquid) 37 stored in the upper liquid space 20 and the liquid (second liquid) 38 stored in the lower liquid space 22 are shown by diagonal lines in different directions. The lower liquid space 22 is arranged below the upper liquid space 20, and the liquid spaces 20 and 22 are connected to each other by the connection pore 26. As shown in FIG. 3, the liquid spaces 20 and 22 intersect each other at a right angle in plan view.

The particle analysis device 1 also includes a hole (first hole) 20A, a hole 20B, a hole (second hole) 22A, and a hole 22B. Each of the holes 20A, 20B, 20C, and 20d has an opening that opens at the top surface of the particle analysis device 1.

The hole 20A and the hole 20B extend vertically from the top surface of the particle analysis device 1 to the upper liquid space 20. The hole 22A and the hole 22B extend vertically from the top surface of the particle analysis device 1 to the lower liquid space 22. The hole 20A, the hole 20B, and the upper liquid space 20 form a reservoir for the liquid 37. The hole 22A, the hole 22B, and the lower liquid space 22 form another reservoir for the liquid 38.

Furthermore, the particle analysis device 1 has a first electrode 28 and a second electrode 30. The first electrode 28 is used for applying an electric potential to the liquid 37 in the first liquid space 20 through the hole 20A. The second electrode 30 is used for applying an electric potential through the hole 22A to the liquid 38 in the lower liquid space 22. The electric potential applied by the second electrode 30 is different from that applied by the first electrode 28. For example, the second electrode 30 is an anode and the first electrode 28 is a cathode. Since the liquid spaces 20 and 22 are connected via the connection pore 26, an electric current flows through the liquid 37 and the liquid 38 inside the liquid spaces 20 and 22.

FIG. 4 schematically illustrates the principle of particle analysis used in the particle analysis device 1. In the upper liquid space 20, the liquid 37 containing particles 40 to be analyzed is stored. In the lower liquid space 22, a liquid 38, which does not originally contain the particles 40, is stored. However, the liquid 38 stored in the lower liquid space 22 may contain the particles 40. The liquid spaces 20 and 22 are connected to each other via the connection pore 26 that is a through-hole formed in a chip (nanopore chip) 24. A DC (direct current) power supply 35 and a current meter 36 are connected to the first electrode 28 and the second electrode 30. The DC power supply 35 is, for example, a battery, but is not limited to a battery.

Electrophoresis caused by the potential difference applied to the electrodes 28 and 30 causes the particles 40 contained in the liquid 37 stored in the lowermost plate 2 to pass the connection pore 26 and to flow into the liquid 38 stored in the lower liquid space 22. When the particles 40 pass through the connection pore 26, the current value flowing through the liquid 37 and the liquid 38 changes. The change in current value can be observed using the current meter 36. By observing the change in the current value, characteristics (e.g., type, shape, and size) of the particles 40 that passed through the connection pore 26 can be analyzed. For example, it is possible to determine the number of particles 40 of a certain type contained in the liquid 37. The particle analysis device 1 can be used to analyze a variety of particles, such as exosomes, pollens, viruses, and bacteria.

As shown in FIGS. 1, 2, and 3, the particle analysis device 1 includes multiple stacked square plates 2, 4, 6, 8, and 10 joined together. Preferably, some or all of these plates are formed from transparent or semi-transparent material, and storage state of the liquid 37 or the liquid 38 in the cavities of the particle analysis device 1 (the hole 20A, the hole 20B, the hole 22A, and the hole 22B, and the liquid spaces 20 and 22) can be observed from outside the particle analysis device 1. However, it is not absolutely necessary that the storage states of the liquids are observable, and these plates may be opaque.

The plates 2, 4, 6, 8, and 10 are formed from electrically and chemically inert and insulating materials. Each plate may be formed from a rigid material or from an elastic material. Preferred rigid materials include resin materials, such as polycarbonate, polyethylene terephthalate, acrylic, cyclic olefin, polypropylene, polystyrene, polyester, and polyvinyl chloride. Preferred elastic materials include elastomers, for example, silicone rubber containing PDMS (polydimethylsiloxane) or urethane rubber.

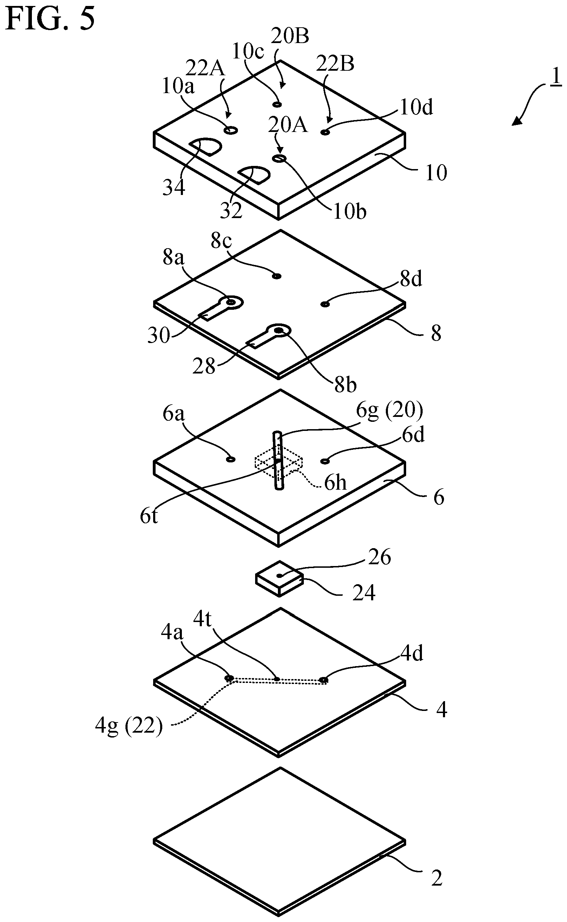

As shown in FIGS. 2 and 5, neither grooves nor holes are formed in the lowermost plate 2. The plate 2 is formed, for example, from one of the preferred rigid materials described above.

A horizontal groove 4g is formed in the center of the lower surface of the next plate 4. When the plates 2 and 4 are joined together, the groove 4g forms the lower liquid space 22. In the center of the groove 4g, a communication hole 4t penetrating the plate 4 in a vertical direction is formed. The communication hole 4t connects the lower liquid space 22 (groove 4g) with the connection pore 26 of the chip 24. In addition, vertically penetrating cylindrical through-holes 4a and 4d are formed in the plate 4. The through-holes 4a and 4d have the same diameter. The through-hole 4a communicates with one end of the groove 4g, whereas the through-hole 4d communicates with the other end of the groove 4g. The plate 4 may be formed from one of the rigid materials described above, but is preferably formed from one of the elastic materials described above.

A recess 6h having a rectangular-parallelepiped shape is formed in the center of the lower surface of the next plate 6. The recess 6h contains the chip 24 having the connection pore 26. The chip 24 is fitted into the recess 6h. The chip 24 may be removable or non-removable from the recess 6h. A horizontal groove 6g is formed in the center of the upper surface of the plate 6. When the plates 6 and 8 are joined together, the groove 6g forms the upper liquid space 20. In the center of the groove 6g, a vertically penetrating communication hole 6t is formed. The communication hole 6t connects the upper liquid space 20 (the groove 6g) with the connection pore 26 of the chip 24. The cross sections of the communication holes 4t and 6t are circular.

The plate 6 has vertically penetrating cylindrical through-holes 6a and 6d. The through-holes 6a and 6d have the same diameter as that of the through-holes 4a and 4d. The through-hole 6a communicates with the through-hole 4a of the plate 4 immediately below it, and thus with one end of the groove 4g, whereas the through-hole 6d communicates with the through-hole 4d, and thus with the other end of the groove 4g. The plate 6 may be formed from one of the rigid materials described above, but is preferably formed from one of the elastic materials described above.

The chip (nanopore chip) 24 has a rectangular parallelepiped shape, for example, a square plate shape. In the center of the chip 24, the vertically penetrating connection pore 26 is formed. The chip 24 is made from an electrically and chemically inert and insulating material, such as glass, sapphire, a ceramic, a resin, an elastomer, SiO₂, SiN, or Al₂O₃. Preferably, the chip 24 is made from a material harder than the material of the plates 2, 4, 6, 8, and 10, for example, glass, sapphire, ceramics, SiO₂, SiN, or Al₂O₃, but a resin or an elastomer may be used to form the chip 24. The user may select an appropriate chip 24 depending on the application of the particle analysis device 1. For example, the user may prepare multiple chips 24 with connection pores 26 having different dimensions or shapes, and may select a chip 24 to be fitted into the recess to change the particles 40 to be analyzed.

It is preferable that the chip 24 be hydrophilized so that the liquid can easily pass through the connection hole 26 without clogging the connection pore 26. The hydrophilization treatment includes, for example, irradiating the chip 24 with oxygen plasma or ultraviolet rays. Ultraviolet rays may be irradiated in the form of a laser beam.

In the next plate 8, cylindrical through-holes 8a, 8b, 8c, and 8d penetrating the plate 8 in a vertical direction are formed. The through-holes 8a, 8b, 8c, and 8d have the same diameter as that of the through-holes 4a, 4d, 6a and 6d. The through-hole 8a communicates with the through-hole 6a of the plate 6 disposed immediately below it, whereas the through-hole 8d communicates with the through-hole 6d. The through-hole 8b communicates with one end of the groove 6g of the plate 6, whereas the through-hole 8c communicates with the other end of the groove 6g. On the upper surface of the plate 8, the electrodes 28 and 30 are arranged in parallel, and the first electrode 28 gives an electric potential to the liquid 37 in the through-hole 8b, whereas the second electrode 30 gives another electric potential to the liquid 38 in the through-hole 8a. The plate 8 may be formed from one of the rigid materials described above, but is preferably formed from one of the elastic materials described above.

In the uppermost plate 10, vertically penetrating through-holes 10a, 10b, 10c, and 10d are formed. The through-holes 10a and 10b have a diameter that is greater than that of the through-holes 8a, 8b, 8c, and 8d, whereas the through-holes 10c and 10d have a diameter that is equal to that of the through-holes 8a, 8b, 8c, and 8d. The through-holes 10a, 10b, 10c, and 10d respectively communicate with the through-holes 8a, 8b, 8c, and 8d of the plate 8 immediately below them.

In addition, in the uppermost plate 10, a first electrode-rod-insertion hole 32 exposing the first electrode 28 disposed below the plate 10 and a second electrode-rod-insertion hole 34 exposing the second electrode 30 are formed. Each of the electrode-rod-insertion holes 32 and 34 has an opening that opens at the top surface of the particle analysis device 1, and penetrates the plate 10 to extend from the top surface to the electrode 28 or 30. The plate 10 may be formed from one of the elastic materials described above, but is formed from one of the rigid materials described above.

An electrode rod is inserted into each of the electrode-rod-insertion holes 32 and 34. The electrode rods are brought into contact with the electrodes 28 and 30, respectively, and apply electric potentials to the liquids 37 and 38.

The aforementioned hole 20A is constituted of the through-holes 10b and 8B and penetrates the plates 10 and 8 to reach one end of the groove 6g in the plate 6, i.e., the upper liquid space 20. The through-hole 10b is the opening of the hole 20A that opens at the top surface of the particle analysis device 1. In the middle of the hole 20A, the first electrode 28 is provided. The hole 20B is constituted of the through-holes 10c and 8c and penetrates the plates 10 and 8 to reach the other end of the groove 6g in the plate 6, i.e., the upper liquid space 20. The through-hole 10c is the opening of the hole 20B that opens at the top surface of the particle analysis device 1.

The hole 22A is constituted of the through-holes 10a, 8a, 6a, and 4a and penetrates the plates 10, 8, 6, and 4 to reach one end of the groove 4g in the plate 4, i.e., the lower liquid space 22. The through-hole 10a is the opening of the hole 22A that opens at the top surface of the particle analysis device 1. The second electrode 30 is provided in the middle of the hole 22A. The hole 22B is constituted of the through-holes 10d, 8d, 6d, and 4d and penetrates the plates 10, 8, 6, and 4 to reach the other end of the groove 4g in the plate 4, i.e., the lower liquid space 22. The through-hole 10d is the opening of the hole 22B that opens at the top surface of the particle analysis device 1.

These plates 2, 4, 6, 8, and 10 can be bonded together with an adhesive. However, in order to prevent or reduce undesirable inflow of organic matter into the liquid spaces 20 and 22, it is preferable to use irradiation of vacuum ultraviolet light or oxygen plasma to join the plates 2, 4, 6, 8, and 10. When joining the plates 2, 4, 6, 8, and 10, it is preferable that the plates 2, 4, 6, 8, and 10 be compressed in a vertical direction, so that leakage of liquid from the holes 20A, 20B, 22A, and 22B and the liquid spaces 20 and 22 is prevented as far as possible after joining.

When the chip 24 is formed from a brittle material, at least one of the plates 4 and 6 around the chip 24 is preferably formed from one of the above-described elastic materials in order to prevent the chip 24 from being damaged. In addition, in order to prevent leakage of liquid in the connection pore 26 of the chip 24, the plate 6, into which the chip 24 is fitted, is preferably formed from one of the above-described elastic materials, and the recess 6h of the plate 6 preferably has dimensions (horizontal dimensions) suitable for the chip 24 to be tightly fitted. Furthermore, in order to prevent a gap from occurring between the lower surface of the chip 24 and the upper surface of the plate 4, the depth of the recess 6h is preferably the same as or slightly greater than the height of the chip 24.

The electrodes 28 and 30 are formed from materials with high electrical conductivity. For example, silver-silver chloride (Ag/AgCI), platinum, or gold can be used to form the electrodes 28 and 30. Alternatively, the electrodes 28 and 30 can be formed from a material containing any or all of these metals and an elastomer.

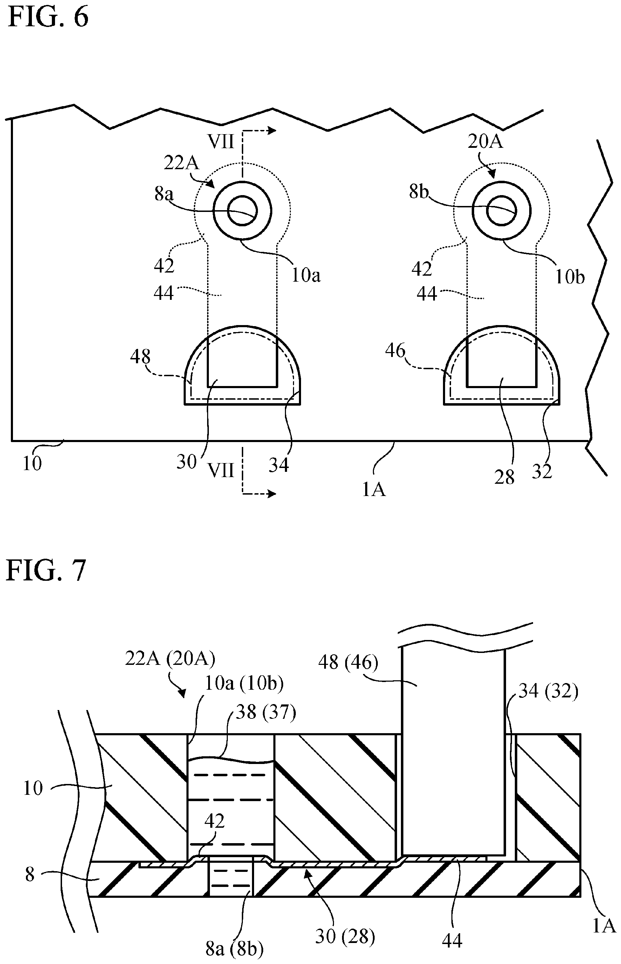

Each of the electrodes 28 and 30 formed on the plate 8 is a flat sheet and is sandwiched between the two plates 8 and 10. As shown in FIG. 6, each of the electrodes 28 and 30 has a circular annular portion 42 formed around the through-hole 8b or 8a (a part of the hole 20A or the hole 22A) of the plate 8 and a rectangular extending portion 44 connected to the circular annular portion 42.

The circular annular portion 42 has a through-hole having a diameter that is the same as those of the through-hole 8a and 8b. The circular annular portion 42 is formed substantially concentrically with the through-hole 8a or 8b of the plate 8, and substantially concentrically overlaps with the through-hole 10a or 10b of the plate 10 directly above.

An end of the extending portion 44 on the opposite side of the circular annular portion 42 overlaps the electrode-rod-insertion hole 32 or 34 of the plate 10 directly above. The width of the extension portion 44 is less than the outer diameter of the circular annular portion 42. A first electrode rod 46 is inserted into the first electrode-rod-insertion hole 32 and is brought into contact with the circular annular portion 42 of the first electrode 28, and the second electrode rod 48 is inserted into the second electrode-rod-insertion hole 34 and is brought into contact with the circular annular portion 42 of the second electrode 30.

The hole 20A has the through-hole 10b, which is disposed above the first electrode 28, and the through-hole 8b, which is disposed below the first electrode 28. The through-hole 10b has a greater diameter and thus a greater area than those of the through-hole 8b. The outer diameter of the circular annular portion 42 of the first electrode 28 is greater than the diameter of the through-hole 10b disposed immediately above it.

The hole 22A has the through-hole 10a, which is disposed above the second electrode 30, and the through-hole 8a, which is disposed below the second electrode 30. The through-hole 10a has a greater diameter and thus a greater area than those of the through-hole 8a. The outer diameter of the circular annular portion 42 of the second electrode 30 is greater than the diameter of the through-hole 10a disposed immediately above it.

Thus, the circular annular portion 42 of each electrode overlaps the through-hole 10b or 10a having an opening area greater than that of the through-hole 8b or 8a. Therefore, the contact area between the liquid injected into the holes and the electrodes is secured to be large, and the reliability of analysis of the particles can be improved. As shown in FIG. 7, the second electrode 30 is in contact with the liquid 38 inside the hole 22A (through-holes 10A and 8A) with a large contact area, and the first electrode 28 is in contact with the liquid 37 inside the hole 20A (through-holes 10b and 8b) with a large contact area.

In addition, since the outer diameter of the circular annular portion 42 is greater than that of the through-holes 10b and 10a immediately above the circular annular portion 42, so that even when the position of the circular annular portion 42 deviates slightly from the desired position (i.e., even when the accuracy of the position of the circular annular portion 42 is incorrect), the circular annular portion 42 overlaps the through-hole 10b or 10a with a high degree of reliability. Accordingly, in a plurality of particle analysis devices 1, the contact area of the liquid injected into the holes and the electrodes is uniform, and the reliability of the particle analysis can be improved.

As shown in FIG. 7, each of the electrodes 28 and 30 intersects a hole 20A or hole 22A approximately orthogonally. Each of the electrodes 28 and 30 is a flat sheet, so that the electrodes 28 and 30 are compressed between the two plates 8 and 10 and are deformed as shown in FIG. 7. Since the plates 8 and 10 are made from a softer material than the material of the electrodes 28 and 30, the plates 8 and 10 also deform and absorb the thickness of the electrodes 28 and 30 (FIG. 7 shows the softer plate 8 is more deformed than the plate 10).

However, due to the thickness of the electrodes 28 and 30, gaps may occur around the electrodes 28 and 30 between the plates 8 and 10. The gaps between the plates 8 and 10 allow the liquid in the holes 20A and 22A to leak out.

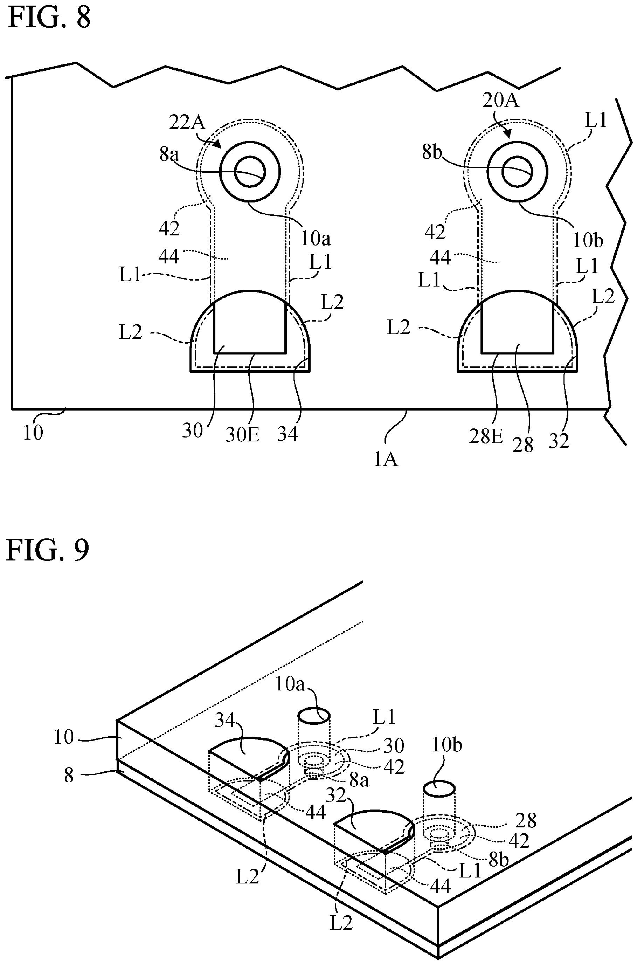

FIGS. 8 and 9 show the liquid leaking from holes 20A and 22A in phantom lines. The liquid that was in contact with the top surface of the circular annular portion 42 of each electrode may leak out into the gaps between the plates 8 and 10 around the electrodes due to the thickness of the electrodes, as liquid leakage L1. The ranges of the liquid leakage L1 correspond to the gaps between the plates 8 and 10 around the electrodes.

The liquid leakage L1 travels from the side of the circular annular portion 42 of the electrode through both sides of the extending portion 44 to reach the electrode-rod-insertion holes 32 or 34, and can appear in the electrode-rod-insertion holes 32 and 34, as liquid leakage L2. The ranges of the liquid leakage L2 are within the electrode-rod-insertion holes 32 and 34 surrounded by the walls of the plates 8 and 10. Around most of the electrode-rod-insertion holes 32 and 34, the electrodes 28 and 30 are not interposed between the plates 8 and 10. Accordingly, there is almost no liquid leakage L2 outside the electrode-rod-insertion holes 32 and 34. That is to say, in this embodiment, the width of the extending portions 44 of the electrodes 28 and 30 is less than the width of the electrode-rod-insertion holes 32 and 34, and the ends 28E and 30E of the extending portions 44 of the electrodes 28 and 30 are disposed within the ranges of the electrode-rod-insertion holes 32 and 34, so that there is almost no liquid leakage L2 outside the electrode-rod-insertion holes 32 and 34.

In the areas in which there is no liquid leakage L1 or L2, the plate 10 and the plate 8 can be regarded as being liquid-tightly bonded. In other words, around the entirety of the first electrode 28, the plate 10 directly above the first electrode 28 and the second electrode 30 is liquid-tightly bonded to the plate 8 directly below the first electrode 28 and the second electrode 30, and around the entirety of the second electrode 30, the plate 10 directly above the first electrode 28 and the second electrode 30 is liquid-tightly bonded to the plate 8 directly below the first electrode 28 and the second electrode 30. In addition, a region in which the plate 10 is liquid-tightly bonded to the plate 8 is interposed between the first electrode 28 and the second electrode 30.

If the liquid leakage reaches the side surface 1A of the particle analysis device 1, then the liquid is likely to travel through the interference between the plates 10 and 8 at the side surface 1A. In this case, the first liquid 37 that was stored in the first hole 20A may come into contact with the liquid 38 that was stored in the second hole 22A via the interference between the plates 10 and 8 at the side surface 1A. In other words, the electrodes 28 and 30, which are sandwiched between the same two plates 10 and 8 and arranged at the same height level, may be short-circuited. If the electrodes 28 and 30 are short-circuited, the current flowing between the electrodes 28 and 30 may become excessive and/or unstable, making it impossible to accurately analyze the particles.

However, each of the electrode-rod-insertion holes 32 and 34 extends from the top surface of the particle analysis device 1 to the corresponding electrode 28 or 30 and does not open at any side surface 1A, 1B, 1C, or 1D of the particle analysis device 1, and the first electrode 28 and the second electrode 30 are not exposed at side surfaces 1A, 1B, 1C, and 1D of the particle analysis device 1. In addition, the electrodes 28 and 30 are located away from any side surface of the particle analysis device 1. The electrode 28 terminates within the electrode-rod-insertion hole 32 and is farther from the side surface 1A of the particle analysis device 1 than an end of the first electrode-rod-insertion hole 32 on the side of the side surface 1A of the particle analysis device 1 (an outer end of the hole 32). The electrode 30 also terminates within the electrode-rod-insertion hole 34 and is farther from the side surface 1A of the particle analysis device 1 than an end of the first electrode-rod-insertion hole 34 on the side of the side surface 1A of the particle analysis device 1 (an outer end of the hole 34). Accordingly, in a case in which the liquid leaks from the inside of the first hole 20A and flows along the first electrode 28 between the two plates 10 and 8, even if the leaking liquid may enter the first electrode-rod-insertion hole 32 surrounded by walls of the plates 10 and 8, the leaking liquid is unlikely to leak from the side surfaces of the particle analysis device 1. Therefore, if the distance between the first electrode 28 and the second electrode 30 is sufficiently large and the distance between the first electrode-rod-insertion hole 32 and the second-electrode-rod insertion hole 34 is sufficiently large, the liquid, which leaked from the inside of the first hole 20A, is less likely to cause a short circuit between the first electrode 28 and the second electrode 30.

On the other hand, in a case in which the liquid leaks from the inside of the second hole 22Aand flows along the second electrode 30 between the two plates 10 and 8, even if the leaking liquid may enter the second electrode-rod-insertion hole 34 surrounded by walls of the plates 10 and 8, the leaking liquid is unlikely to leak from the side surfaces of the particle analysis device 1. Therefore, if the distance between the first electrode 28 and the second electrode 30 is sufficiently large and the distance between the first electrode-rod-insertion hole 32 and the second-electrode-rod insertion hole 34 is sufficiently large, the liquid, which leaked from the inside of the second hole 22A, is less likely to cause a short circuit between the first electrode 28 and the second electrode 30.

Thus, according to the particle analysis device 1, it is possible to improve the certainty of particle analysis.

Furthermore, in a region away from the first electrode 28, the plate 10 directly above the first electrode 28 and the second electrode 30 is liquid-tightly bonded to the plate 8 directly below the first electrode 28 and the second electrode 30, around the entirety of the first electrode 28. In a region away from the second electrode 30, the plate 10 directly above the first electrode 28 and the second electrode 30 is liquid-tightly bonded to the plate 8 directly below the first electrode 28 and the second electrode 30, around the entirety of the second electrode 30. In addition, a region in which the plate 10 is liquid-tightly bonded to the plate 8 is interposed between the first electrode 28 and the second electrode 30. Accordingly, even if the liquid leaks from the inside of the first hole 20A and flows along the first electrode 28 between the two plates, the liquid is unlikely to reach the second electrode 30, and even if the liquid leaks from the inside of the second hole 22 and flows along second electrode 30 between the two plates, the liquid is unlikely to reach the first electrode 28. Therefore, the risk of the liquid causing a short circuit between the first and second electrodes 28 and 30 is reduced.

In a case in which the particle analysis device 1 is stored, it is preferable to place the particle analysis device 1 in a vacuum or underwater environment to protect the above-mentioned hydrophilized chip 24 from the air. However, in a case in which the particle analysis device 1 is stored in an underwater environment, water that has entered the holes 20A and 22A causes the liquid leakage L1 and L2. Even if the particle analysis device 1 is taken out from the water for use and the surfaces of the particle analysis device 1 are wiped with a cloth, it is difficult to remove the liquid leakage L1 and L2 between two plates 10 and 8. In this case, if the liquid leakage is connected between the electrodes 28 and 30, the electrodes 28 and 30 will be short-circuited.

In this embodiment, however, the risk of a short circuit between the electrodes 28 and 30 is small because of the reasons mentioned above. Therefore, according to this particle analysis device 1, it is possible to improve the certainty of particle analysis.

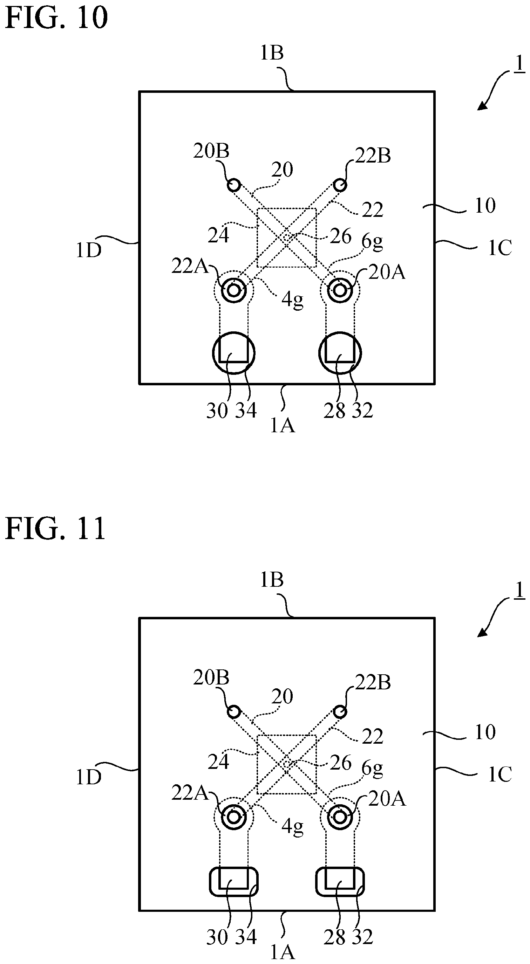

In this embodiment, the electrode-rod-insertion holes 32 and 34 have an approximately semicircular contour. However, the contours of the electrode-rod-insertion holes 32 and 34 are not limited to the shape in the embodiment. The electrode-rod-insertion holes 32 and 34 may have a circular contour, as shown in FIG. 10, or a rectangular contour, as shown in FIG. 11.

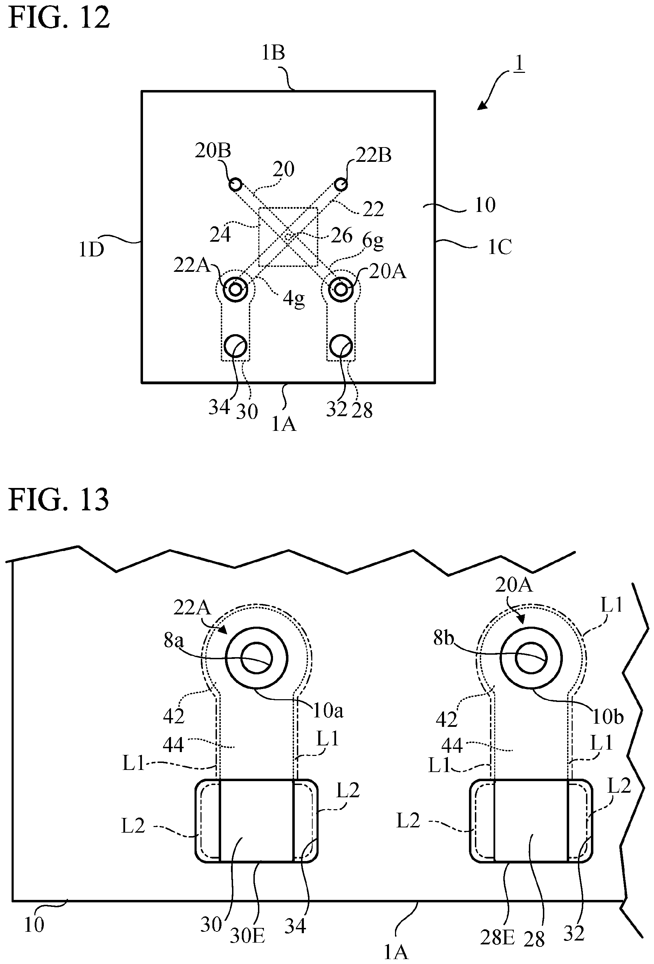

Furthermore, the width of the electrode-rod-insertion holes 32 and 34 in the embodiment is greater than the width of the extending portions 44 of the electrodes 28 and 30. However, the relative size of the electrode-rod-insertion holes 32 and 34 to the extending portions 44 of the electrodes 28 and 30 is not limited to the embodiment. As shown in FIG. 12, the width of the electrode-rod-insertion holes 32 and 34 may be less than the width of the extending portions 44 of the electrodes 28 and 30, and the electrode-rod-insertion holes 32 and 34 fall within the ranges of the ends of the extending portions 44. In this case, even if liquid leakage occurs in the gaps between the plates 8 and 10 around the electrodes 28 and 30, there is a small risk that the liquid will cause a short circuit between the first electrode 28 and the second electrode 30 if the distance between the first electrode 28 and the second electrode 30 is sufficiently large and the distance between the first electrode-rod-insertion hole 32 and the second-electrode-rod insertion hole 34 is sufficiently large

Furthermore, in the embodiment, as shown in Fig. 8, the electrodes 28 and 30 terminate within the electrode-rod-insertion holes 32 and 34, respectively, and the ends 28E and 30E of the electrodes 28 and 30 are distant respectively from the inner walls of the electrode-rod-insertion holes 32 and 34 on the side of the side surface 1A. However, as shown in FIG. 13, the ends 28E and 30E of the electrodes 28 and 30 may be respectively in contact with the inner walls of the electrode-rod-insertion holes 32 and 34 on the side of the side surface 1A. Even in this case, there is almost no liquid leakage L2 outside the electrode-rod-insertion holes 32 and 34.

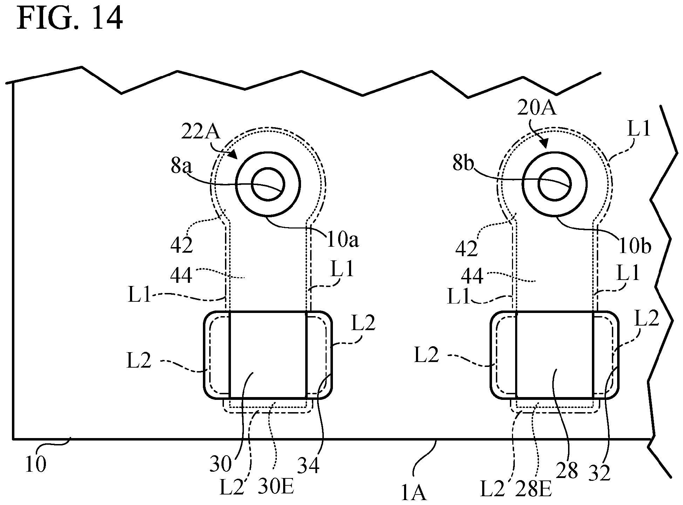

In addition, as shown in Fig. 14, the ends 28E and 30E of the electrodes 28 and 30 may extend toward the side surface 1A beyond the inner walls of the electrode-rod-insertion holes 32 and 34 on the side of the side surface 1A, respectively. However, the ends 28E and 30E do not reach the side surface 1A of the particle analysis device 1 and are not exposed at the side surface 1A. As described above, the plates 10 and 8 are liquid-tightly bonded by means of irradiation of vacuum ultraviolet light or oxygen plasma. Even if there is a small amount of liquid leakage L2 outside the electrode-rod-insertion holes 32 and 34, the liquid leakage L2 will not reach the side surface 1A if there is a sufficiently large distance between the side surface 1A and the ends 28E and 30E of the electrodes 28 and 30.

The present invention has been illustrated and described with reference to a preferred embodiment of the present invention. However, it will be understood that a person skilled in the art can change the form and details without departing from the scope of the invention described in the claims. It is intended that such changes, modifications, and alterations should fall within the scope of the present invention.

For example, a compression mechanism (e.g., a clamping mechanism, screws, or a pinch) that constantly compresses the particle analysis device in a vertical direction may be used to improve sealing between the plates of the particle analysis device.

The number of plates in the particle analysis device is not limited to the above embodiment. In the embodiment, the upper liquid space 20 is defined by the groove 6g formed in the single plate 6, but the upper liquid space 20 may be defined by multiple plates (e.g., plates 6 and 8). In the embodiment, the lower liquid space 22 is defined by the groove 4g formed on the single plate 4, but the lower liquid space 22 may be defined by multiple plates (e.g., plates 4 and 2). In the embodiment, the chip 24 having the connection pore 26 is located inside the single plate 6, but the chip 24 may be disposed inside multiple plates (e.g., plates 6 and 4).

In the embodiment, the extending portions 44 of the electrodes 28 and 30 are rectangular and has a uniform width. However, the extending portions 44 may have wider and narrower portions, or the width of the extending portions 44 may gradually decrease or increase toward the side surface 1A.

Aspects of the present invention are also set out in the following numbered clauses:
Clause 1. A particle analysis device including:
   multiple stacked plates joined together;
   an upper liquid space formed in at least one of the plates and adapted to store a first liquid;
   a lower liquid space formed in at least one of the plates, disposed below the upper liquid space and adapted to store a second liquid;
   a connection pore disposed in at least one of the plates and connecting the upper liquid space to the lower liquid space;
   a first hole having an opening that opens at a top surface of the particle analysis device, the first hole penetrating at least one of the plates and extending from the top surface to the upper liquid space, the first liquid flowing through the first hole;
   a second hole having an opening that opens at the top surface, the second hole penetrating at least two of the plates and extending from the top surface to the lower liquid space, the second liquid flowing through the second hole;
   a first electrode that is a sheet pinched between two of the plates and adapted to apply an electric potential to the first liquid in the upper liquid space through the first hole;
   a second electrode that is a sheet pinched between the two of the plates, between which the first electrode is pinched, and adapted to apply an electric potential to the second liquid in the lower liquid space through the second hole;
   a first electrode-rod-insertion hole having an opening that opens at the top surface, the first electrode-rod-insertion hole penetrating at least one of the plates and extending from the top surface to the first electrode; and
   a second electrode-rod-insertion hole having an opening that opens at the top surface, the second electrode-rod-insertion hole penetrating at least one of the plates and extending from the top surface to the second electrode,
   the first electrode and the second electrode being configured not to be exposed at a side surface of the particle analysis device.
Clause 2. The particle analysis device according to clause 1, wherein the first electrode and the second electrode are located away from a side surface of the particle analysis device.
Clause 3. The particle analysis device according to clause 1 or 2, wherein the first electrode terminates within the first electrode-rod-insertion hole and is farther from a side surface of the particle analysis device than an outer end of the first electrode-rod-insertion hole, and wherein the second electrode terminates within the second electrode-rod-insertion hole and is farther from the side surface of the particle analysis device than an outer end of the second electrode-rod-insertion hole.
Clause 4. The particle analysis device according to clause 3, wherein the first electrode-rod-insertion hole has an inner wall on a side of the side surface, wherein the first electrode has an end that is distant from the inner wall of the first electrode-rod-insertion hole, wherein the second electrode-rod-insertion hole has an inner wall on the side of the side surface, and wherein the second electrode has an end that is distant from the inner wall of the second electrode-rod-insertion hole.
Clause 5. The particle analysis device according to clause 3, wherein the first electrode-rod-insertion hole has an inner wall on a side of the side surface, wherein the first electrode has an end that is in contact with the inner wall of the first electrode-rod-insertion hole, wherein the second electrode-rod-insertion hole has an inner wall on the side of the side surface, and wherein the second electrode has an end that is in contact with the inner wall of the second electrode-rod-insertion hole.
Clause 6. The particle analysis device according to clause 1 or 2, wherein the first electrode-rod-insertion hole has an inner wall on a side of the side surface, wherein the first electrode has an end that extends toward the side surface beyond the inner wall of the first electrode-rod-insertion hole, wherein the second electrode-rod-insertion hole has an inner wall on the side of the side surface, and wherein the second electrode has an end that extends toward the side surface beyond the inner wall of the second electrode-rod-insertion hole.

Clause 4. The particle analysis device according to any one of clauses 1 to 6, wherein a plate directly above the first electrode and the second electrode is liquid-tightly bonded to a plate directly below the first electrode and the second electrode around the entirety of the first electrode, wherein the plate directly above the first electrode and the second electrode is liquid-tightly bonded to the plate directly below the first electrode and the second electrode around the entirety of the second electrode, and wherein a region in which the plate directly above the first electrode and the second electrode is liquid-tightly bonded to the plate directly below the first electrode and the second electrode is interposed between the first electrode and the second electrode.

According to this clause, even if the liquid leaks from the inside of the first hole and flows along the first electrode between the two plates, the liquid is unlikely to reach the second electrode, and even if the liquid leaks from the inside of the second hole and flows along second electrode between the two plates, the liquid is unlikely to reach the first electrode. Therefore, the risk of the liquid causing a short circuit between the first and second electrodes is reduced.

### REFERENCE SYMBOLS

1: Particle analysis device
1A, 1B, 1C, 1D: Side surface
2, 4, 6, 8, 10: Plate
20: Upper liquid space
22: Lower liquid space
24: Chip (nanopore chip)
26: Connection pore
37: Liquid (first liquid)
38: Liquid (second liquid)
20A: Hole (first hole)
20B: Hole
22A: Hole (second hole)
22B: Hole
28: First electrode
30: Second electrode
32: First electrode-rod-insertion hole
34: Second electrode-rod-insertion hole
46: First electrode rod
48: Second electrode rod
L1: Liquid leakage
L2: Liquid leakage

## Claims

1. A particle analysis device comprising:
multiple stacked plates joined together;
an upper liquid space formed in at least one of the plates and adapted to store a first liquid;
a lower liquid space formed in at least one of the plates, disposed below the upper liquid space and adapted to store a second liquid;
a connection pore disposed in at least one of the plates and connecting the upper liquid space to the lower liquid space;
a first hole having an opening that opens at a top surface of the particle analysis device, the first hole penetrating at least one of the plates and extending from the top surface to the upper liquid space, the first liquid flowing through the first hole;
a second hole having an opening that opens at the top surface, the second hole penetrating at least two of the plates and extending from the top surface to the lower liquid space, the second liquid flowing through the second hole;
a first electrode that is a sheet pinched between two of the plates and adapted to apply an electric potential to the first liquid in the upper liquid space through the first hole;
a second electrode that is a sheet pinched between the two of the plates, between which the first electrode is pinched, and adapted to apply an electric potential to the second liquid in the lower liquid space through the second hole;
a first electrode-rod-insertion hole having an opening that opens at the top surface, the first electrode-rod-insertion hole penetrating at least one of the plates and extending from the top surface to the first electrode; and
a second electrode-rod-insertion hole having an opening that opens at the top surface, the second electrode-rod-insertion hole penetrating at least one of the plates and extending from the top surface to the second electrode,
the first electrode and the second electrode being configured not to be exposed at a side surface of the particle analysis device.

2. The particle analysis device according to claim 1, wherein the first electrode and the second electrode are located away from a side surface of the particle analysis device.

3. The particle analysis device according to claim 1 or 2, wherein the first electrode terminates within the first electrode-rod-insertion hole and is farther from a side surface of the particle analysis device than an outer end of the first electrode-rod-insertion hole, and wherein the second electrode terminates within the second electrode-rod-insertion hole and is farther from the side surface of the particle analysis device than an outer end of the second electrode-rod-insertion hole.

4. The particle analysis device according to claim 3, wherein the first electrode-rod-insertion hole has an inner wall on a side of the side surface, wherein the first electrode has an end that is distant from the inner wall of the first electrode-rod-insertion hole, wherein the second electrode-rod-insertion hole has an inner wall on the side of the side surface, and wherein the second electrode has an end that is distant from the inner wall of the second electrode-rod-insertion hole.

5. The particle analysis device according to claim 3, wherein the first electrode-rod-insertion hole has an inner wall on a side of the side surface, wherein the first electrode has an end that is in contact with the inner wall of the first electrode-rod-insertion hole, wherein the second electrode-rod-insertion hole has an inner wall on the side of the side surface, and wherein the second electrode has an end that is in contact with the inner wall of the second electrode-rod-insertion hole.

6. The particle analysis device according to claim 1 or 2, wherein the first electrode-rod-insertion hole has an inner wall on a side of the side surface, wherein the first electrode has an end that extends toward the side surface beyond the inner wall of the first electrode-rod-insertion hole, wherein the second electrode-rod-insertion hole has an inner wall on the side of the side surface, and wherein the second electrode has an end that extends toward the side surface beyond the inner wall of the second electrode-rod-insertion hole.

7. The particle analysis device according to any one of claims 1 to 6, wherein a plate directly above the first electrode and the second electrode is liquid-tightly bonded to a plate directly below the first electrode and the second electrode around the entirety of the first electrode, wherein the plate directly above the first electrode and the second electrode is liquid-tightly bonded to the plate directly below the first electrode and the second electrode around the entirety of the second electrode, and wherein a region in which the plate directly above the first electrode and the second electrode is liquid-tightly bonded to the plate directly below the first electrode and the second electrode is interposed between the first electrode and the second electrode.
